# FASCICULE DE BREVET EUROPEEN

(11) **EP 1 162 096 B1**
(45) Date de publication et mention de la délivrance du brevet: **10.09.2008**
(21) Numéro de dépôt: 01401481.5
(22) Date de dépôt: 08.06.2001
(51) Int. Cl.: B60H 3/06, B60H 3/00, A61L 9/12

(54) **Dispositif de filtration destiné à équiper un appareil d'aération et/ou de chauffage et/ou de climatisation, notamment pour véhicule automobile**
Filtervorrichtung für ein Gerät zur Belüftung und/oder zur Beheizung und/oder zur Klimatisierung, insbesondere für Kraftfahrzeuge
Filtration device for an apparatus for ventilating and/or heating and/or cooling, especially for a motor vehicle

(30) Priorité: 08.06.2000 FR 0007348
(43) Date de publication de la demande: 12.12.2001
(73) Titulaire: Valeo Matériaux de Friction, 87020 Limoges cedex 9 (FR)
(72) Inventeur: Adamczak, Loic, 87110 Bosmie l'Aiguille (FR); Robert, Samuel, 50240 Saint-James (FR)
(74) Mandataire: Lemaire, Marc

(56) Documents cités:
- DE-A- 2 825 171
- FR-A- 2 794 405
- GB-A- 1 182 595
- GB-A- 2 021 435
- US-A- 4 953 449

## Description

La présente invention a pour objet un dispositif de filtration destiné à équiper un appareil d'aération et/ou de chauffage et/ou de climatisation, notamment pour véhicule automobile.

Dans un véhicule automobile l'appareil à l'intérieur duquel est monté le dispositif de filtration comporte au moins une partie d'entrée dotée d'au moins un ventilateur. Cette partie d'entrée est utilisée pour prendre de l'air venant de l'extérieur, qui - en aval du ventilateur, en variante en amont du ventilateur- est destiné à traverser le dispositif de filtration. En aval de cette partie d'entrée il est prévu un radiateur de chauffage en communication avec l'intérieur du véhicule. En variante comme visible dans le document EP-A- 0 634 299 il est prévu un dispositif de climatisation doté d'un évaporateur placé en aval du dispositif de filtration. Dans cette configuration la partie d'entrée de l'appareil précité est également utilisée pour prendre de l'air venant de l'intérieur du véhicule.

Le dispositif de filtration permet donc de filtrer l'air pollué venant de l'extérieur et/ou de l'intérieur du véhicule. Ce dispositif comporte au moins un cadre et un médiat filtrant.

On a déjà proposé dans les documents GB-A- 1 182 595 (divulguant toutes les caractéristiques du préambule de la revendication 1), DE-A- 28 25 171 et FR-A- 2 794 405 (non publié à la date de priorité de la présente demande) de doter un tel dispositif de filtration d'une fonction odorisation. Plus précisément dans le document GB-A- 1 182 595 il est prévu des poches portant un média d'adsorption traité avec du parfum et/ou un désinfectant en sorte que la durée de vie d'un tel dispositif d'odorisation n'est pas aussi longue que souhaitée le parfum étant libéré lors du stockage du filtre. Dans le document FR-A- 2 749 405 on pulvérise un extrait de parfum liquide ou d'huile essentielle sur le filtre en sorte que le dispositif d'odorisation est libéré tout de suite après pulvérisation.

Dans le document DE-A- 28 25 171 il est prévu un dispositif d'odorisation sous la forme d'aiguilles remplies de déodorant et enfoncées dans le média filtrant du dispositif de filtration. Il est possible de remplacer les aiguilles.

Cette disposition n'est pas applicable lorsque le média filtrant est de faible épaisseur, notamment lorsqu'il est du type plissé. En outre on peut perdre des aiguilles lors de la manipulation, du conditionnement ou du transport du dispositif de filtration.

La présente invention a pour objet de doter, de manière fiable simple et économique, un tel dispositif de filtration à média filtrant de faible épaisseur d'une fonction d'odorisation.

Ainsi, selon l'invention, un dispositif de filtration destiné à équiper un appareil d'aération et/ou de chauffage et/ou de climatisation, notamment pour un véhicule automobile, du genre comportant au moins un cadre, un média filtrant, notamment du type plissé, et un dispositif d'odorisation, est caractérisé par le fait que le dispositif d'odorisation consiste en des micro-capsules d'odorisation associées au cadre et/ou au média filtrant.

Avantageusement, le dispositif de filtration comportant également au moins une grille ; selon l'invention les micro-capsules d'odorisation sont associées à la grille.

Toutes les combinaisons sont possibles.

Les micro-capsules contiennent dans un mode de réalisation du parfum, en variante les micro-capsules contiennent une huile essentielle. En variante les micro-capsules contiennent également un désinfectant tel qu'un anti-bactériens. En variante des micro-capsules de désinfectant sont ajoutées au micro-capsules déodorantes.

Au cours de l'utilisation du dispositif de filtration les membranes, usuellement en matière plastique, des micro-capsules sont érodées soit par l'air, soit par les particules de l'air, ce qui libère progressivement la matière odorante de celles-ci par exemple du parfum.

Une odeur agréable est ainsi générer dans l'habitacle du véhicule automobile tout au long de la durée de vie du média filtrant.

Ainsi grâce à la présence des membranes la fonction odorisation a une plus grande durée de vie que si l'on plongeait le dispositif de filtration ou l'un au moins des composants de celui-ci dans un bain rempli d'une matière odorante.

En outre la quantité de matière odorante utilisée est plus réduite grâce aux micro-capsules qui ont par définition une faible taille autorisant un passage de l'air notamment à travers le média filtrant.

Ces micro-capsules sont associées, c'est à dire liées par tous moyens connus, à au moins un des composants du dispositif de filtration en sorte que le média filtrant peut être dans tous les cas du type plissé.

Dans tous les cas avant utilisation du dispositif de filtration, par exemple lors de manipulations, du transport et/ou du conditionnement du média filtrant, contrairement aux aiguilles de l'art antérieur, on ne risque pas de perdre les micro-capsules en sorte que la solution est fiable.

De préférence, le média filtrant comprend un mat de non tissé constitué d'un voile de carde.

Dans une forme de réalisation, ledit mat de non tissé constitué d'un voile de carde est appliqué contre ladite grille sans lui être solidarisé. En variante le voile de carde est solidarisé à la grille.

De préférence, ledit voile de carde forme une nappe unique.

Avantageusement, ledit voile de carde est placé en amont de la grille en considérant le sens normal de passage du gaz à filtrer.

De préférence, le dispositif de filtration comporte des moyens d'adsorption de gaz placés entre la grille et ledit mat de non tissé.

Avantageusement, lesdits moyens d'adsorption comportent un tissu de carbone activé.

Avantageusement, lesdits moyens d'adsorption comportent un lit de charbon activé.

De préférence, un mat de non tissé constitué d'un voile de carde est disposé de part et d'autre des moyens d'adsorption.

Avantageusement, une seconde grille, dite grille d'entrée, est disposée en amont du mat de non tissé.

De préférence, les deux grilles sont de même nature et sont dans une forme de réalisation solidarisées au média filtrant.

De préférence, le mat de non tissé est à base de fibres longues dont la longueur est, de préférence, au moins égale à 38 mm.

Avantageusement, lesdites fibres longues présentent un diamètre moyen au moins égal à 1 µm.

De préférence, lesdites fibres longues sont en matériau thermoplastique.

Avantageusement, lesdites fibres longues sont des fibres de polypropylène.

Avantageusement, la grille est réalisée en polypropylène.

De préférence, le cadre est en polypropylène.

Avantageusement, les micro-capsules sont déposées par tous moyens connus, par exemple par pulvérisation, à la surface du média filtrant et/ou du cadre et/ou de la grille.

En variante, les micro-capsules sont introduites dans la masse de matière(s) à partir de laquelle sont réalisés le média filtrant et/ou la grille et/ou le cadre.

Plus précisément, le dispositif de filtration comporte une grille souple avantageusement plissée contre laquelle est disposé, sans lui être solidarisé, un média filtrant formant filtre à particules, formé d'un mat de non tissé constitué d'un voile de carde en une nappe unique. Le média filtrant est lui-même plissé de la même manière que la grille. En variante la grille est solidaire du média filtrant par exemple par une soudure par ultrasons. Ainsi dans une première étape on superpose le média filtrant en nappe et au moins une grille en matière thermoplastique, puis on procède dans une seconde étape à la fixation de la grille et du média filtrant par une soudure par ultrasons et enfin on réalise une étape de plissage de ces deux éléments superposés. L'étape de soudage est dans un mode de réalisation réalisée à l'aide d'une sonotrode sur la même machine que l'étape de plissage. En variante l'étape de fixation consiste en un calandrage à chaud de ces éléments disposés entre rouleaux ou calandre sur toute leur surface. Aucune désolidarisation ne peut ainsi se produire en cas de chocs, de chutes ou de vibrations lors de la manipulation, du conditionnement ou du transport.

Ledit mat de non tissé est à base de fibres longues dont la longueur est, de préférence, au moins égale à 38 mm. Lesdites fibres longues présentent un diamètre moyen au moins égal à 1 µm. Lesdites fibres sont en matériau thermoplastique et, ici, en polypropylène.

La grille est en matière thermoplastique, ici en polypropylène. Elle se trouve placée en aval par rapport au sens de circulation de l'air dans le dispositif de filtration.

Une seconde grille plissée et souple peut être prévue en sorte que le média filtrant se trouve placé entre les grilles sans être solidarisé ni à l'une ni à l'autre des deux grilles. En variante le média filtrant est solidarisé aux deux grilles de manière précitée par exemple par un soudage à ultrasons.

De préférence, la seconde grille dite d'entrée est placée en amont du média selon le sens de circulation de l'air dans le dispositif de filtration.

Avantageusement, la seconde grille est de même nature que la première grille souple et est ici en polypropylène.

En variante, les deux grilles sont de natures différentes.

Le dispositif de filtration constitue un filtre à particules.

L'invention concerne aussi un filtre dit combiné, c'est-à-dire à la fois un filtre à particules et un filtre arrêtant des gaz délétères.

Dans ce cas, à la structure de base constituée de la grille aval et du média, sont ajoutés des moyens d'adsorption de gaz placés entre la grille et le média en sorte d'avoir successivement, dans le sens de circulation de l'air, le média, les moyens d'adsorption de gaz et la grille en aval.

Un filtre combiné comporte avantageusement successivement, dans le sens de circulation de l'air, une grille d'entrée, un média, des moyens d'adsorption de gaz, un média et une grille de sortie. Les médias sont avantageusement de la même nature de celle décrite précédemment. De la sorte, deux mats de non tissé constitués chacun d'un voile de carde sont disposés respectivement de part et d'autre des moyens d'adsorption de gaz.

Lesdits moyens d'adsorption de gaz peuvent comporter un lit de charbon activé, mais, de préférence, ils comprennent un tissu de carbone plissé de la même manière que la grille et que le média.

Lorsque le dispositif de filtration présente une construction symétrique, celle-ci permet avantageusement son montage indifféremment dans un sens ou dans l'autre dans un appareil de chauffage et/ou ventilation et/ou climatisation, ce qui rend inutile de prévoir, dans ce cas, un détrompeur pour le sens de montage. Le dispositif de filtration est ainsi réversible. Bien entendu, dans ce cas, la grille d'entrée peut jouer le rôle de grille de sortie et, inversement, la grille de sortie jouer le rôle de grille d'entrée.

Un cadre est prévu pour supporter les différents constituants du dispositif de filtration.

Le cadre est réalisé par exemple en matière thermoplastique, ici en polypropylène, et est solidarisé à la grille de sortie ou à la grille d'entrée, et de préférence aux deux grilles quand toutes les deux sont prévues.

La solidarisation d'une grille au cadre s'effectue avantageusement par une opération dite de soudure miroir qui consiste à chauffer simultanément jusqu'au début de fusion une partie périphérique de la grille, d'une part, et une portion du cadre correspondant à la zone de fixation, d'autre part, puis à rapprocher et serrer l'une contre l'autre cette partie périphérique et cette portion de manière à exercer une pression relative.

L'opération de soudure miroir est conduite de telle manière que l'extrémité latérale de la grille soit amenée à se plier à angle droit pour épouser la face interne correspondante du cadre, ce qui a pour avantage d'assurer une meilleure fixation de la grille et d'éviter toute fuite d'air entre grille et cadre.

Avantageusement, plusieurs parties périphériques et portions correspondantes, de la grille et du cadre, sont ainsi fixées entre elles.

La même nature chimique, ici du polypropylène, de la grille et du cadre permet une fixation solide et durable.

Lors de l'opération de soudure miroir, le ou les mats de non tissé sont apposés contre la ou les grilles correspondantes avec, le cas échéant, interposition de la manière décrite précédemment, de moyens d'adsorption tels que, de préférence, un tissu de carbone.

En variante, le cadre est moulé autour de la grille ou des deux grilles ainsi que sur le ou les médias filtrants.

Un procédé préférentiel de fabrication d'un dispositif de filtration est le suivant.

Des fibres de polypropylène d'une finesse de 1,7 dtex (diamètre 14 µm) coupées à une longueur de 40 mm sont introduites dans un mélangeur.

Le mélange de fibres obtenu est ensuite introduit dans une démoteuse puis dans un silo de stockage avant d'être cardé au moyen d'une carde de type laine à double peigneur équipée d'un brouilleur de type pêle-mêle afin d'obtenir un voile de carde isotrope.

Le voile est, en sortie de carde, introduit dans un condenseur pour former une nappe.

Les paramètres de la carde et du condenseur sont choisis de manière à obtenir une nappe présentant une masse surfacique d'environ 20 à 150 g/m².

La nappe est déposée sur un tapis puis consolidée par une opération de liage hydraulique.

L'opération de liage hydraulique consiste à faire traverser la nappe disposée sur un tambour rotatif par des jets d'eau très fins sous haute pression, comprise entre 40 et 200 bars.

En rebondissant sur le tambour, les jets d'eau provoquent un nouage des fibres conférant à la nappe une résistance mécanique importante sans pour autant en augmenter, de manière conséquente, la densité.

Après passage dans un tunnel de séchage, la nappe est enroulée.

La nappe est ensuite reprise pour subir une opération de plissage et se trouve alors prête à être montée sur un support pour former le dispositif de filtration comme indiqué précédemment.

Le dispositif de filtration comporte une nappe en une seule couche ; de la sorte, avec une nappe de 0,84 mm d'épaisseur, on a pu former un filtre présentant une masse surfacique de 66 g/m² et de densité 0,07.

Pour tester l'efficacité du filtre ainsi réalisé, on a placé ce dernier en travers d'une veine de section égale à 100 cm², parcourue par un courant d'air chargé de particules à une vitesse de 20 cm/s.

Les particules correspondent à une poudre dite "poudre SAE fine" qui est définie par la norme des Etats-Unis d'Amérique, référence SAE J 726/ISO 5011.

La concentration de particules est de 100 mg/m³.

On constate, d'une part, que la perte de charge au travers du filtre n'est, dans ces conditions, que de 14 Pa et que, d'autre part, l'efficacité atteint 80 %.

Par rapport aux dispositifs de filtration actuellement sur le marché, on a obtenu une efficacité comparable aux meilleurs d'entre eux avec un produit d'une densité beaucoup plus faible (0,07 contre 0,12 à 0,30) et engendrant une perte de charge nettement plus faible.

Des tests d'endurance ont en outre démontré une plus grande stabilité dans le temps de l'efficacité du dispositif de filtration.

Pour doter un tel dispositif de filtration d'une fonction odorisation, on dépose des micro-capsules contenant une matière odorante, ici un parfum, à la surface des fibres du média filtrant ici après le passage de la nappe dans le tunnel de séchage. Cette disposition se combine bien avec le filtre précité à faible épaisseur et faible perte de charge.

Au cours de l'utilisation du filtre, les membranes des micro-capsules sont érodées et le parfum est libéré progressivement; une odeur agréable est générée. Grâce à l'invention, l'odorisation est obtenue sans qu'il soit nécessaire d'ajouter un odorisant, sous forme de plaquette ou autre, dans le local ou l'habitacle.

En variante les micro-capsules sont déposées à la surface du cadre et/ou de la grille.

L'invention prévoit également l'introduction des micro-capsules dans la masse de matière(s) à partir de laquelle sont réalisées les fibres du média filtrant et/ou la grille et/ou le cadre.

Par exemple de manière aisée on dépose les micro-capsules sur le cadre ou on introduit les micro-capsules dans le cadre du document EP-A- 0 634 299 au niveau de l'ouverture centrale de celui-ci en regard du média filtrant et/ou au niveau des ouvertures de by-pass de celui-ci.

Lorsque les micro-capsules sont introduites dans le cadre ou déposées sur le cadre on minimise les pertes de charge puisque le média filtrant n'est pas affecté par les micro-capsules.

Lorsque les micro-capsules sont associées au cadre et au média filtrant, le média filtrant est affecté totalement par les micro-capsules ou en variante partiellement par exemple au voisinage du cadre pour réduire les pertes de charge.

L'association des micro-capsules avec la grille est aisée à réaliser car cette grille comporte des barres espacées s'étendant longitudinalement et/ou verticalement par rapport au média filtrant en sorte que l'on peut choisir les barres qui seront associées aux micro-capsules. La localisation des micro-capsules est donc plus précise et plus facile à réaliser que sur le média filtrant. Bien entendu la deuxième grille en variante porte des micro-capsules odorantes.

Dans ce qui précède, le média filtrant comprend un mat de non tissé mais, comme on le comprend aisément, l'invention s'applique quel que soit le type de média filtrant qui peut comprendre, par exemple, un voile de carde consolidé par liage hydraulique associé audit mat ; en variante, il s'agit d'un complexe fibreux comprenant deux couches de non-tissé entre lesquelles est disposée une grille de renforcement, l'ensemble étant solidarisé par interpénétration des deux couches de non-tissé.

De même, s'agissant du cadre, celui-ci peut être un encadrement total ou partiel réalisé par collage, soudage, surmoulage ou autre, amovible ou non.

## Revendications

1. Dispositif de filtration destiné à équiper un appareil d'aération et/ou de chauffage et/ou de climatisation, notamment pour un véhicule automobile, du genre comportant au moins un cadre, un média filtrant, notamment du type plissé, et un dispositif de d'odorisation, **caractérisé par le fait que** le dispositif d'odorisation consiste en des micro-capsules associées au cadre et/ou au média filtrant.

2. Dispositif de filtration destiné à équiper un appareil d'aération et/ou de chauffage et/ou de climatisation, notamment pour un véhicule automobile, du genre comportant au moins un cadre, un média filtrant, notamment du type plissé, un dispositif de d'odorisation et au moins une grille, **caractérisé par le fait que** le dispositif d'odorisation consiste en des micro-capsules associées à la grille.

3. Dispositif de filtration selon la revendication 1 ou 2, **caractérisé par le fait que** les micro-capsules contiennent un parfum et éventuellement un désinfectant et en ce que les micro-capsules sont associées au cadre et/ou au média filtrant et/ou à la grille.

4. Dispositif de filtration selon l'une des revendications 1 à 3, **caractérisé par le fait que** le média filtrant comprend un mat de non tissé constitué d'un voile de carde appliqué contre la grille en étant solidarisé à celle-ci.

5. Dispositif de filtration selon l'une des revendications 1 à 3, **caractérisé par le fait que** le média filtrant comprend un mat de non tissé constitué d'un voile de carde appliqué contre ladite grille sans lui être solidarisé.

6. Dispositif de filtration selon l'une des revendications 4 ou 5, **caractérisé par le fait que** ledit voile de carde forme une nappe unique.

7. Dispositif de filtration selon l'une des revendications 2 à 6, **caractérisé par le fait que** ledit voile de carde est placé en amont de la grille en considérant le sens normal de passage du gaz à filtrer.

8. Dispositif de filtration selon l'une des revendications 2 à 7, **caractérisé par le fait qu'**il comporte des moyens d'adsorption de gaz placés entre la grille et ledit mat de non tissé.

9. Dispositif de filtration selon la revendication 8, **caractérisé par le fait que** lesdits moyens d'adsorption comportent un tissu de carbone activé.

10. Dispositif de filtration selon la revendication 8, **caractérisé par le fait que** lesdits moyens d'adsorption comportent un lit de charbon activé.

11. Dispositif de filtration selon l'une des revendications 8 à 10, **caractérisé par le fait qu'**un mat de non tissé constitué d'un voile de carde est disposé de part et d'autre des moyens d'adsorption.

12. Dispositif de filtration selon l'une des revendications 2 à 11 prises en combinaison avec la revendication 2, **caractérisé par le fait qu'**une seconde grille, dite grille d'entrée, est disposée en amont du mat de non tissé.

13. Dispositif de filtration selon la revendication 12, **caractérisé par le fait que** les deux grilles sont de même nature.

14. Dispositif de filtration selon l'une quelconque des revendications 4 à 13, **caractérisé par le fait que** le mat de non tissé est à base de fibres longues dont la longueur est, de préférence, au moins égale à 38 mm.

15. Dispositif de filtration selon la revendication 14, **caractérisé par le fait que** lesdites fibres longues présentent un diamètre moyen au moins égal à 1µm.

16. Dispositif de filtration selon l'une des revendications 14 ou 15, **caractérisé par le fait que** lesdites fibres longues sont en matériau thermoplastique.

17. Dispositif de filtration selon l'une des revendications 14 à 16, **caractérisé par le fait que** lesdites fibres longues sont des fibres de polypropylène.

18. Dispositif de filtration selon l'une des revendications 2 à 17, **caractérisé par le fait que** la grille est réalisée en polypropylène.

19. Dispositif de filtration selon l'une des revendications 1 à 18, **caractérisé par le fait que** le cadre est en polypropylène.

20. Dispositif de filtration selon l'une des revendications 2 à 19, **caractérisé par le fait que** les micro-capsules sont déposées à la surface du média filtrant et/ou du cadre et/ou de la grille.

21. Dispositif de filtration selon l'une des revendications 2 à 19, **caractérisé par le fait que** les micro-capsules sont introduites dans la masse de matière(s) à partir de laquelle sont réalisés le média filtrant et/ou la grille et/ou le cadre.

## Claims

1. Filtering device designed to equip a ventilation and/or heating and/or air-conditioning system, in particular for a motor vehicle, of the type comprising at least a frame, a filtering medium, in particular of the folded type, and a fragrance device, **characterised in that** the fragrance device consists of micro-capsules associated with the frame and/or the filtering medium.

2. Filtering device designed to equip a ventilation and/or heating and/or air-conditioning system, in particular for a motor vehicle, of the type comprising at least a frame, a filtering medium, in particular of the folded type, a fragrance device and at least one grid, **characterised in that** the fragrance device consists of micro-capsules associated with the grid.

3. Filtering device according to claim 1 or claim 2, **characterised in that** the micro-capsules contain a perfume and optionally a disinfectant, and **in that** the micro-capsules are associated with the frame and/or with the filtering medium and/or with the grid.

4. Filtering device according to one of claims 1 to 3, **characterised in that** the filtering medium comprises a mat of non-woven fabric consisting of a web which is applied against the grid and is rendered integral with it.

5. Filtering device according to one of claims 1 to 3, **characterised in that** the filtering medium comprises a mat of non-woven fabric consisting of a web which is applied against the said grid without being rendered integral with it.

6. Filtering device according to one of claims 4 or 5, **characterised in that** the said web forms a single sheet.

7. Filtering device according to one of claims 2 to 6, **characterised in that** the said web is placed upstream from the grid, considering the normal direction of passage of the gas to be filtered.

8. Filtering device according to one of claims 2 to 7, **characterised in that** it comprises means for absorption of gas placed between the grid and the said non-woven mat.

9. Filtering device according to claim 8, **characterised in that** the said adsorption means comprise an active carbon fabric.

10. Filtering device according to claim 8, **characterised in that** the said adsorption means comprise an active carbon bed.

11. Filtering device according to one of claims 8 to 10, **characterised in that** a mat of non-woven fabric constituted by a web is disposed on both sides of the adsorption means.

12. Filtering device according to one of claims 2 to 11 taken in combination with claim 2, **characterised in that** a second grid, known as the intake grid, is disposed upstream from the mat of non-woven fabric.

13. Filtering device according to claim 12, **characterised in that** the two grids are of the same type.

14. Filtering device according to one of claims 4 to 13, **characterised in that** the mat of non-woven fabric is based on long fibres, the length of which is preferably at least equal to 38 mm.

15. Filtering device according to claim 14, **characterised in that** the said long fibres have an average diameter at least equal to 1µm.

16. Filtering device according to one of claims 14 or 15, **characterised in that** the said long fibres are made of thermoplastic material.

17. Filtering device according to one of claims 14 to 16, **characterised in that** the said long fibres are polypropylene fibres.

18. Filtering device according to one of claims 2 to 17, **characterised in that** the grid is made of polypropylene.

19. Filtering device according to one of claims 1 to 18, **characterised in that** the frame is made of polypropylene.

20. Filtering device according to one of claims 2 to 19, **characterised in that** the micro-capsules are deposited on the surface of the filtering medium and/or the frame and/or the grid.

21. Filtering device according to one of claims 2 to 19, **characterised in that** the micro-capsules are introduced into the mass of material(s) from which the filtering medium and/or the grid and/or the frame are made.

## Patentansprüche

1. Filtervorrichtung zur Ausrüstung einer Belüftungs-, und/oder Heizungs- und/oder Klimaanlage, insbesondere für ein Kraftfahrzeug, mit wenigstens einem Rahmen, einem Filtermedium, insbesondere in gefalteter Ausführung, und einer Geruchsverbesserungsvorrichtung, **dadurch gekennzeichnet, dass** die Geruchsverbesserungsvorrichtung aus mit dem Rahmen und/oder mit dem Filtermedium verbundenen Mikrokapseln besteht.

2. Filtervorrichtung zur Ausrüstung einer Belüftungs-, und/oder Heizungs- und/oder Klimaanlage, insbesondere für ein Kraftfahrzeug, mit wenigstens einem Rahmen, einem Filtermedium, insbesondere in gefalteter Ausführung, einer Geruchsverbesserungsvorrichtung und wenigstens einem Gitter, **dadurch gekennzeichnet, dass** die Geruchsverbesserungsvorrichtung aus mit dem Gitter verbundenen Mikrokapseln besteht.

3. Filtervorrichtung nach Anspruch 1 oder 2, **dadurch gekennzeichnet, dass** die Mikrokapseln einen Duftstoff und gegebenenfalls ein Desinfektionsmittel enthalten und dass die Mikrokapseln mit dem Rahmen und/oder mit dem Filtermedium und/oder mit dem Gitter verbunden sind.

4. Filtervorrichtung nach einem der Ansprüche 1 bis 3, **dadurch gekennzeichnet, dass** das Filtermedium eine Vliesmatte umfasst, die aus einem Krempelvlies besteht, das gegen das Gitter angedrückt wird, wobei es mit diesem fest verbunden ist.

5. Filtervorrichtung nach einem der Ansprüche 1 bis 3, **dadurch gekennzeichnet, dass** das Filtermedium eine Vliesmatte umfasst, die aus einem Krempelvlies besteht, das gegen das besagte Gitter angedrückt wird, ohne fest mit ihm verbunden zu sein.

6. Filtervorrichtung nach einem der Ansprüche 4 oder 5, **dadurch gekennzeichnet, dass** das besagte Krempelvlies eine einheitliche Lage bildet.

7. Filtervorrichtung nach einem der Ansprüche 2 bis 6, **dadurch gekennzeichnet , dass** das besagte Krempelvlies vor dem Gitter mit Blick auf die normale Strömungsrichtung des zu filternden Gases angeordnet ist.

8. Filtervorrichtung nach einem der Ansprüche 2 bis 7, **dadurch gekennzeichnet, dass** sie Gasadsorptionsmittel umfasst, die zwischen dem Gitter und der besagten Vliesmatte angeordnet sind.

9. Filtervorrichtung nach Anspruch 8, **dadurch gekennzeichnet, dass** die besagten Adsorptionsmittel ein Aktivkohlegewebe umfassen.

10. Filtervorrichtung nach Anspruch 8, **dadurch gekennzeichnet , dass** die besagten Adsorptionsmittel ein Aktivkohlebett umfassen.

11. Filtervorrichtung nach einem der Ansprüche 8 bis 10, **dadurch gekennzeichnet , dass** eine aus einem Krempelvlies bestehende Vliesmatte beiderseits der Adsorptionsmittel angeordnet ist.

12. Filtervorrichtung nach einem der Ansprüche 2 bis 11 in Kombination mit Anspruch 2, **dadurch gekennzeichnet, dass** ein als Einlassgitter bezeichnetes zweites Gitter vor der Vliesmasse angeordnet ist.

13. Filtervorrichtung nach Anspruch 12, **dadurch gekennzeichnet, dass** die beiden Gitter gleichartig ausgeführt sind.

14. Filtervorrichtung nach einem der Ansprüche 4 bis 13, **dadurch gekennzeichnet, dass** die Vliesmatte auf der Basis von Langfasern ausgeführt ist, deren Länge vorzugsweise mindestens gleich 38 mm ist.

15. Filtervorrichtung nach Anspruch 14, **dadurch gekennzeichnet, dass** die besagten Langfasern einen mittleren Durchmesser mindestens gleich 1 µm aufweisen.

16. Filtervorrichtung nach einem der Ansprüche 14 oder 15, **dadurch gekennzeichnet, dass** die besagten Langfasern aus Thermoplast bestehen.

17. Filtervorrichtung nach einem der Ansprüche 14 bis 16, **dadurch gekennzeichnet, dass** die besagten Langfasern Polypropylenfasern sind.

18. Filtervorrichtung nach einem der Ansprüche 2 bis 17, **dadurch gekennzeichnet, dass** das Gitter aus Polypropylen ausgeführt ist.

19. Filtervorrichtung nach einem der Ansprüche 1 bis 18, **dadurch gekennzeichnet, dass** der Rahmen aus Polypropylen besteht.

20. Filtervorrichtung nach einem der Ansprüche 2 bis 19, **dadurch gekennzeichnet, dass** die Mikrokapseln an der Oberfläche des Filtermediums und/oder des Rahmens und/oder des Gitters angeordnet sind.

21. Filtervorrichtung nach einem der Ansprüche 2 bis 19, **dadurch gekennzeichnet, dass** die Mikrokapseln in die Werkstoffmasse eingebracht sind, aus der das Filtermedium und/oder das Gitter und/oder der Rahmen ausgeführt sind.
